# EUROPEAN PATENT SPECIFICATION

(11) **EP 2 200 610 B1**
(45) Date of publication and mention of the grant of the patent: **10.01.2018**
(21) Application number: 08831500.7
(22) Date of filing: 19.09.2008
(51) Int. Cl.: A61K 31/4468, A61P 25/00

(54) **CO-ADMINISTRATION OF PIMAVANSERIN WITH OTHER AGENTS**
COADMINISTRATION VON PIMAVANSERIN MIT ANDEREN MITTELN
CO-ADMINISTRATION DE PIMAVANSÉRINE AVEC D'AUTRES AGENTS

(30) Priority: 21.09.2007 US 974426 P; 07.11.2007 US 986250 P; 06.05.2008 US 50976
(43) Date of publication of application: 30.06.2010
(73) Proprietor: Acadia Pharmaceuticals Inc., San Diego, CA 92139 (US)
(72) Inventor: HACKSELL, Uli, Tampa, FL 33639 (US); MCFARLAND, Krista, Walnut Creek, CA 94597 (US)
(74) Representative: Savic, Bojan
(86) International application number: PCT/US2008/077139
(87) International publication number: WO 2009/039460

(56) References cited:
- EP-A1- 1 576 985
- WO-A-2004/064738
- WO-A-2005/112927
- US-A1- 2005 261 340

## Description

### BACKGROUND

### Field

The present application relates to the fields of chemistry and medicine. More particularly, disclosed herein are methods and compositions that can be used to treat disease conditions such as those characterized by cholinergic abnormalities.

### Description of the Related Art

Conditions associated with cognitive impairment, such as Alzheimer's disease, are accompanied by loss of acetylcholine in the brain. This is believed to be the result of degeneration of cholinergic neurons in the basal forebrain, which widely innervate multiple areas of the brain, including the association cortices and hippocampus that are critically involved in higher processes.

Efforts to increase acetylcholine levels have focused on increasing levels of choline, the precursor for acetylcholine synthesis, and on blocking acetylcholinesterase (AChE), the enzyme that metabolizes acetylcholine. To date, attempts to augment central cholinergic function through the administration of choline or phosphatidylcholine have not been successful. AChE inhibitors have shown therapeutic efficacy, but have been found to have frequent cholinergic side effects due to excessive increases in acetylcholine in the periphery-mediated and central-mediated, including abdominal cramps, nausea, vomiting, and diarrhea. These gastrointestinal side effects have been observed in about a third of the patients treated. In addition, some AChE inhibitors, such as tacrine, have also been found to cause significant hepatotoxicity with elevated liver transaminases observed in about 30% of patients. Consequently, the adverse effects of AChE inhibitors have severely limited their clinical utility.

Attempts to ameliorate the effects of decreased acetylcholinergic transmission by direct agonism of the muscarinic M₁ subtype of acetylcholine receptor has not proven successful because known muscarinic agonists lack specificity in their actions at the various muscarinic receptor subtypes, leading to dose-limiting side effects that limit their clinical utility. For example, the M₁ muscarinic agonist arecoline has been found to be an agonist of M₂ as well as M₃ muscarinic receptor subtypes, and is not very effective in treating cognitive impairment, most likely because of dose-limiting M₂ and M₃ receptor mediated side effects. Similarly, xanomeline (Shannon et al., J. Pharmacol. Exp. Ther. 1994, 269, 271; Shannon et al., Schizophrenia Res. 2000, 42, 249) is an M₁/M₄ preferring muscarinic receptor agonist shown to reduce psychotic behavioral symptoms in Alzheimer's disease patients (Bodick et al., Arch. Neurol. 1997, 54, 465), which also suffers from treatment induced side effects that severely limit its clinical utility.

### SUMMARY

One embodiment described herein relates to a composition that can include: pimavanserin, or a salt, a solvate, a polymorph or an isolated, substantially pure metabolite thereof; and an agent that ameliorates one or more cholinergic abnormalities. In some embodiments, the agent that ameliorates one or more cholinergic abnormalities can be a cholinesterase inhibitor. In an embodiment, the cholinesterase inhibitor can be selected from an acetylcholinesterase inhibitor and a butyrylcholinesterase inhibitor. Exemplary cholinesterase inhibitors include, but are not limited to, metrifonate, physostigmine, neostigmine, pyridostigmine, ambenonium, demarcarium, rivastigmine, aldicarb, bendiocarb, bufencarb, carbaryl, carbendazim, carbetamide, carbofuran, chlorbufam, chloropropham, ethiofencarb, formetanate, methiocarb, methomyl, oxamyl, phenmedipham, pinmicarb, pirimicarb, propamocarb, propham, propoxur, galantamine, donepezil (E-2020), tacrine, edrophonium, phenothiazines, echothiophate, diisopropyl fluorophosphate, dimebon, Huperzine A, T-82 ((2-[2-(1-benzylpiperidin-4-yl)ethyl]-2,3-dihydro-9-methoxy-1H-pyrrolo[3,4-b]quinolin-1-one hemifumarate)), TAK-147 (zanapezil), phenserine, quilostigmine, ganstigmine, butyrophenones, imipramines, tropates, phencyclidines, curariforms, ethephon, ethopropazine, iso-OMPA, tetrahydrofurobenzofuran cymserine, N¹phenethyl-norcymserine, N⁸-benzylnorcymserine, N¹, N⁸-bisnorcymserine , N¹-N⁸-bisbenzylnorphysostigmine, N¹,N⁸-bisbenzylnorphenserine and N¹,N⁸-bisbenzylnorcymserine. In an embodiment, the cholinesterase inhibitor can be tacrine.

In other embodiments, the agent that ameliorates one or more cholinergic abnormalities can be a muscarinic receptor agonist. Examples of suitable muscarinic receptor agonists include, but are not limited to, xanomeline, carbamylcholine, oxotremorine, methacholine, bethanechol, cevimeline (AF102B), AF150(S), AF267B, aceclidine, arecoline, milameline, talsaclidine, pilocarpine and (S)-2-ethyl-8-methyl-1-thia-4,8-diazaspiro[4.5]decan-3-one (Torrey Pines NGX267). In an embodiment, the muscarinic receptor agonist can be xanomeline.

In still other embodiments, the agent that ameliorates one or more cholinergic abnormalities can be a glutamatergic antagonist. Suitable glutamatergic antagonists include, but are not limited to, amantadine, dextromethorphan, dextrorphan, ibogaine, ketamine, tramadol, methadone, and memantine. In an embodiment, the glutamatergic antagonist can be memantine.

In some embodiments, the agent that ameliorates one or more cholinergic abnormalities can be a cholinergic agonist. Exemplary cholinergic agonists include, but are not limited to, pramiracetam, piracetam, oxiracetam, choline-L-alfoscerate, nebracetam, besipirdine, and taltirelin.

In other embodiments, the agent that ameliorates one or more cholinergic abnormalities can be a carnitine acetyltransferase stimulant. Exemplary carnitine acetyltransferase stimulants include, but are not limited to, levocarnitine, ST-200 (acetyl-1-carnitine), and nefiracetam.

In still other embodiments, the agent that ameliorates one or more cholinergic abnormalities can be an acetylcholine release stimulant. Exemplary acetylcholine release stimulants include, but are not limited to, SIB-1553A ((+/-)-4-[[2-(1-methyl-2-pyrrolidinyl)ethyl]thio]phenol hydrochloride) and T-588 ((1*R*)-1-benzo [b] thiophen-5-yl-2-[2-(diethylamino) ethoxy] ethan-1-ol hydrochloride).

In even other embodiments, the agent that ameliorates one or more cholinergic abnormalities can be a choline uptake stimulant. In an embodiment, the choline uptake stimulant can be MKC-231 (2-(2-oxopyrrolidin-1-yl)-N-(2,3-dimethyl-5,6,7,8-tetrahydrofuro [2,3-b]quinolin-4-yl)acetoamide).

In some embodiments, the agent that ameliorates one or more cholinergic abnormalities can be a nicotinic acetylcholine receptor agonist. Examples of suitable nicotinic acetylcholine receptor agonist include, but are not limited to, nicotine, ABT-418, ABT-089, SIB-1508Y, A-582941, DMXB-A, Sazetidine-A, Varenicline and TC-1734.

In some embodiments, the agent that ameliorates one or more cholinergic abnormalities can be a 5-HT6 antagonist and/or 5-HT6 inverse agonist. Suitable of 5-HT6 antagonists and/or 5-HT6 inverse agonists include, but are not limited to, SB -742457, SB-271046, SB-399885, SB-357134, SB-258585, RO-436854, RO-0406790 and RO-65-7674.

Another embodiment described herein relates to a method for ameliorating or treating a disease condition characterized by one or more cholinergic abnormalities that can include administering a therapeutically effective amount of one or more compositions described herein to a subject suffering from the disease condition characterized by one or more cholinergic abnormalities.

Still another embodiment described herein relates to a method for ameliorating or treating a disease condition characterized by one or more cholinergic abnormalities that can include administering a therapeutically effective amount of pimavanserin in combination with a therapeutically effective amount of an agent that ameliorates one or more cholinergic abnormalities, such as those described herein, to a subject suffering from the disease condition characterized by one or more cholinergic abnormalities. In an embodiment, pimavanserin can be administered before the agent. In another embodiment, the pimavanserin can be administered after the agent. In still another embodiment, the pimavanserin can be administered at approximately the same time as the agent. Exemplary disease conditions include, but are not limited to, a neuropsychiatric disorder, a neurodegenerative disorder and an extrapyrimidal disorder. Other examples of suitable disease conditions include, but are not limited to, cognitive impairment, forgetfulness, confusion, memory loss, an attention deficit disorder, depression, pain, a sleep disorder, psychosis, a hallucination, aggressiveness, and paranoia. In an embodiment, the psychosis can be selected from drug-induced psychosis, treatment-induced psychosis and psychosis associated with a disease. In some embodiments, the disease the psychosis is associated with can be dementia, post traumatic stress disorder, Alzheimer's disease, Parkinson's disease and schizophrenia. In an embodiment, the psychosis can be Alzheimer's disease-induced psychosis. In an embodiment, the psychosis can be schizophrenia-induced psychosis. In an embodiment, the psychosis can be dementia-related psychosis. Still other exemplary disease conditions include but are not limited to, a neurodegenerative disease, Alzheimer's disease, Parkinson's disease, Huntington's chorea, Friederich's ataxia, Gilles de la Tourette's syndrome, Down Syndrome, Pick disease, dementia, clinical depression, age-related cognitive decline, attention-deficit disorder, sudden infant death syndrome, and glaucoma. In an embodiment, the disease condition can be Alzheimer's disease. In some embodiments, the disease condition can be a cognitive disorder.

### BRIEF DESCRIPTION OF THE DRAWINGS

Figure 1 is a graph that illustrates the results of an amphetamine-induced hyperactivity study and illustrates distance traveled as a function of tacrine dosage.
Figure 2 is a graph that illustrates the results of an amphetamine-induced hyperactivity study and illustrates percent inhibition as a function of tacrine dosage.
Figure 3 is a graph that illustrates the results of an amphetamine-induced hyperactivity study and illustrates distance traveled as a function of xanomeline dosage.
Figure 4 is a graph that illustrates the results of an amphetamine-induced hyperactivity study and illustrates percent inhibition as a function of xanomeline dosage.
Figure 5 is a bar graph that illustrates the results of a novel object recognition study and illustrates the percentage of time spent exploring a novel object as a function of tacrine dosage.
Figure 6 is a bar graph that illustrates the results of a novel object recognition study and illustrates the percentage of time spent exploring a novel object in relation to the drug or combination of drugs administered.
Figure 7 is a graph that illustrates the results of an amphetamine-induced hyperactivity study and illustrates distance traveled as a function of time.
Figure 8 is a graph that illustrates the results of an amphetamine-induced hyperactivity study and illustrates distance traveled as a function of time.
Figure 9 is a graph that illustrates the results of an amphetamine-induced hyperactivity study M₁ knock-out mice and illustrates distance traveled as a function of pimavanserin dosage.
Figure 10 is a graph that illustrates the results of an amphetamine-induced hyperactivity study in M₁ knock-out mice and illustrates percent inhibition as a function of pimavanserin dosage.

### DETAILED DESCRIPTION

Unless defined otherwise, all technical and scientific terms used herein have the same meaning as is commonly understood by one of ordinary skill in the art.

It is understood that, in any compound described herein having one or more chiral centers, if an absolute stereochemistry is not expressly indicated, then each center may independently be of R-configuration or S-configuration or a mixture thereof. Thus, the compounds provided herein may be enatiomerically pure or be stereoisomeric mixtures. In addition it is understood that, in any compound described herein having one or more double bond(s) generating geometrical isomers that can be defined as E or Z each double bond may independently be E or Z a mixture thereof. Likewise, all tautomeric forms are also intended to be included.

An "agonist" is defined as a compound that increases the basal activity of a receptor (i.e. signal transduction mediated by the receptor).

An "inverse agonist" is defined as a compound that decreases the basal activity of a receptor (i.e., signaling mediated by the receptor). Such compounds are also known as negative antagonists. An inverse agonist is a ligand for a receptor that causes the receptor to adopt an inactive state relative to a basal state occurring in the absence of any ligand. Thus, while an antagonist can inhibit the activity of an agonist, an inverse agonist is a ligand that can alter the conformation of the receptor in the absence of an agonist. The concept of an inverse agonist has been explored by Bond et al. in Nature 374:272 (1995). More specifically, Bond et al. have proposed that ligand free β₂-adrenoceptor exists in an equilibrium between an inactive conformation and a spontaneously active conformation. Agonists are proposed to stabilize the receptor in an active conformation. Conversely, inverse agonists are believed to stabilize an inactive receptor conformation. Thus, while an antagonist manifests its activity by virtue of inhibiting an agonist, an inverse agonist can additionally manifest its activity in the absence of an agonist by inhibiting the spontaneous conversion of an unliganded receptor to an active conformation.

As used herein, "antagonist" refers to a compound that competes with an agonist or inverse agonist for binding to a receptor, thereby blocking the action of an agonist or inverse agonist on the receptor. An antagonist attenuates the action of an agonist on a receptor. However, an antagonist (also known as a "neutral agonist") has no effect on constitutive receptor activity. An antagonist may bind reversibly or irreversibly, and may reduce the activity of the receptor until the antagonist is metabolized or dissociates or is otherwise removed by a physical or biological process.

The terms "pure," "purified," "substantially purified," and "isolated" as used herein refer to the compound of the embodiment being free of other, dissimilar compounds with which the compound, if found in its natural state, would be associated in its natural state. In some embodiments described as "pure," "purified," "substantially purified," or "isolated" herein, the compound may comprise at least 75%, 80%, 85%, 90%, 95%, 99% of the mass, by weight, of a given sample.

Pimavanserin, which is also known as N-(1-methylpiperidin-4-yl)-N-(4-fluorophenylmethyl)-N'-(4-(2-methylpropyloxy)phenylmethyl)carbamide, N-[(4-fluorophenyl)methyl]-N-(1-methyl-4-piperidinyl)-N'-[[4-(2-methylpropoxy)phenyl]methyl]-urea, 1-(4-fluorobenzyl)-1-(1-methylpiperidin-4-yl)-3-[4-(2-methylpropoxy)benzyl]urea, or ACP-103 has the structure of Formula (I):

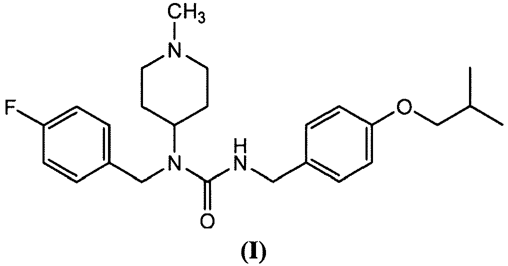

Pimavanserin exhibits activity at serotonin receptors, and acts as an inverse agonist of the 5-HT2A receptor. Experiments performed on cells transiently expressing the human phenotype of the 5-HT2A receptor have shown that pimavanserin attenuates the signaling of such receptors in the absence of additional ligands acting upon the receptor. Pimavanserin has thus been found to possess inverse agonist activity at the 5-HT2A receptor and is able to attenuate the basal, non-agonist-stimulated, constitutive signaling responses that this receptor displays. The observation that pimavanserin is an inverse agonist of the 5-HT2A receptor also indicates that it has the ability to antagonize the activation of 5-HT2A receptors that is mediated by endogenous agonists or exogenous synthetic agonist ligands. Pimavanserin exhibits high affinity for the 5-HT2A receptor with a pKᵢ > 9*. In vivo* human and non-human animal studies have further shown that pimavanserin exhibits antipsychotic, anti-dyskinesia, and anti-insomnia activity. Such properties of pimavanserin are described in U.S. Patent Publication No. 2004-0213816, filed January 15, 2004 and entitled, "SELECTIVE SEROTONIN 2A/2C RECEPTOR INVERSE AGONISTS AS THERAPEUTICS FOR NEURODEGENERATIVE DISEASES,".

Pimavanserin exhibits selective activity at the 5-HT2A receptor. Specifically, pimavanserin lacks functional activity (pEC₅₀ or pKᵢ < 6) at 31 of the 36 human monoaminergic receptors including 5-HT1A, 5-HT1B, 5-HT1D, 5-HT1E, 5-HT1F, 5-HT2B, 5-HT3, 5-HT4, 5-HT6A, 5-HT7A, adrenergic-α1A, adrenergic-α1B, adrenergic-α1D, adrenergic-α2A, adrenergic-α2B, adrenergic-β2, dopamine-D1, dopamine-D2, dopamine-D3, dopamine-D4, histamine-H1, histamine-H2, and histamine-H3. Thus, pimavanserin provides high affinity at 5-HT2A receptors with little to no affinity to most other monoaminergic receptors. In one embodiment, pimavanserin exhibits a pKᵢ of less than 6 to dopamine receptors including the D2 and D4 receptors.

In addition, pimavanserin exhibits high stability, good oral bioavailability, and a long half-life. Specifically, pimavanserin exhibited a slow clearance rate from *in vitro* human microsomes (< 10 µL/min·mg) and a half-life of approximately 55 hours upon oral administration to humans.

Various forms of pimavanserin can be used in the composition with the agent that ameliorates one or more cholinergic abnormalities. For example, a number of salts and crystalline forms of pimavanserin can be used. Exemplary salts include the tartrate, hemi-tartrate, citrate, fumarate, maleate, malate, phosphate, succinate, sulphate, and edisylate (ethanedisulfonate) salts. Pimavanserin salts including the aforementioned ions, among others, are described in U.S. Patent Publication No. 2006-0111399, filed September 26, 2005 and entitled "SALTS OF N-(4-FLUOROBENZYL)-N-(1-METHYLPIPERIDIN-4-YL)-N'-(4-(2-METHYLPROPYLOXY)PHENYLMETHYL)CARBAMIDE AND THEIR PREPARATION,". Two crystalline forms of the tartrate salt are referred to as crystalline Form A and Form C, respectively, and are described in U.S. Patent Publication No. 2006-0106063, filed September 26, 2005 and entitled "SYNTHESIS OF N-(4-FLUOROBENZYL)-N-(1-METHYLPIPERIDIN-4-YL)-N'-(4-(2-METHYLPROPYLOXY)PHENYLMETHYL)CARBAMIDE AND ITS TARTRATE SALT AND CRYSTALLINE FORMS,". Pimavanserin (including, for example, the tartrate salt) may be formulated into tablets, such as is described in more detail in U.S. Patent Publication Nos. 2007-0260064, filed May 15, 2007 and 2007-0264330, filed May 15, 2007, each entitled "PHARMACEUTICAL FORMULATIONS OF PIMAVANSERIN,".

Similarly, isolated, substantially pure metabolites of pimavanserin can also be used. Suitable metabolites that can be used have the chemical structures of Formulae (II), (III), (IV), (V) and (VI) shown below.

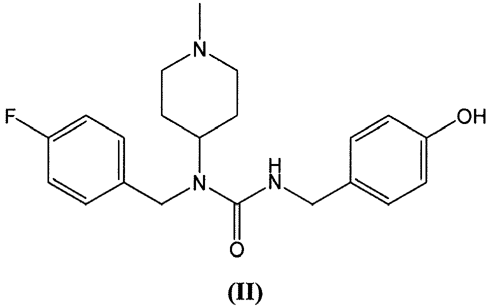

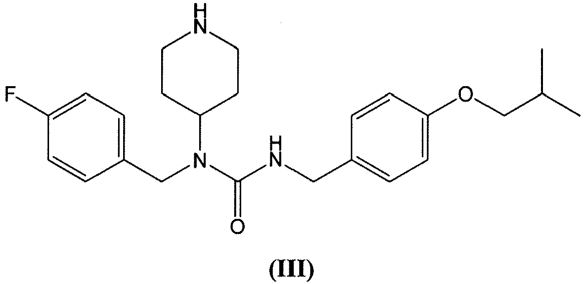

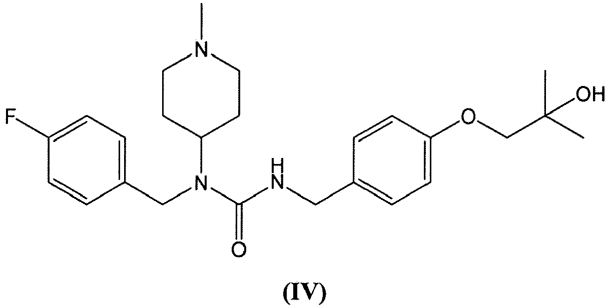

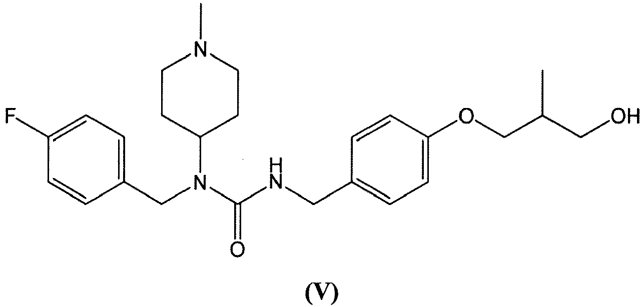

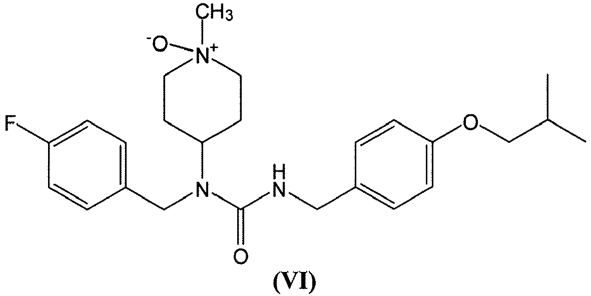

Compounds of Formulae (II), (III), (IV), (V) and (VI) as described herein may be prepared in various ways. General synthetic routes to the compounds of Formulae (II), (III), (IV), (V) and (VI) are shown in Schemes A-E. The routes shown are illustrative only and are not intended, nor are they to be construed, to limit the scope of this invention in any manner whatsoever. Those skilled in the art will be able to recognize modifications of the disclosed synthesis and to devise alternate routes based on the disclosures herein; all such modifications and alternate routes are within the scope of this invention.

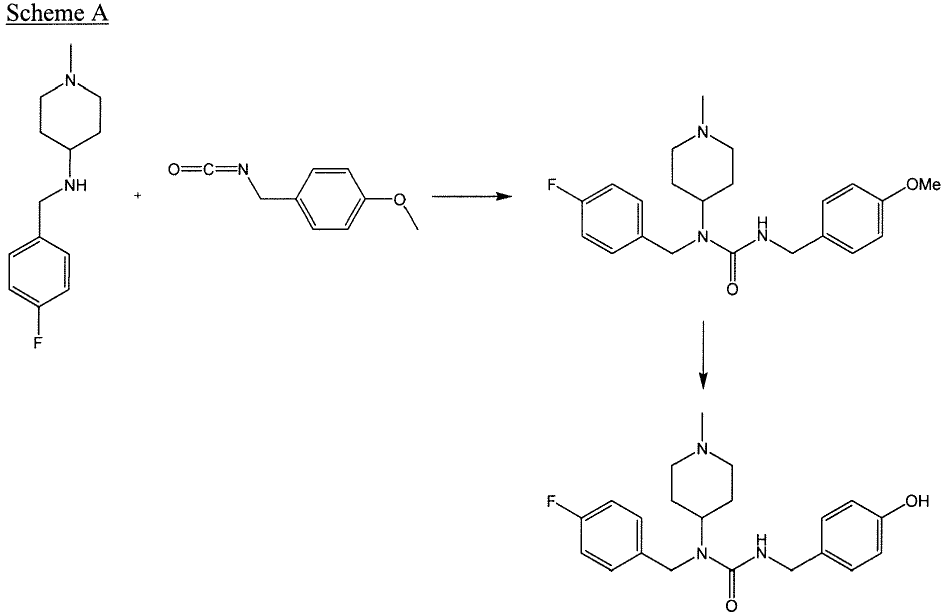

Scheme A shows a general reaction scheme for forming the compound of Formula (II). As shown in Scheme A, the secondary amine and isocyanate can be combined to produce the 4-methoxybenzyl derivative of the compound of Formula (II). The methoxy group can be converted to a hydroxy group using methods known to those skilled in the art, for example, using a boron trihalide to form the compound of Formula (II).

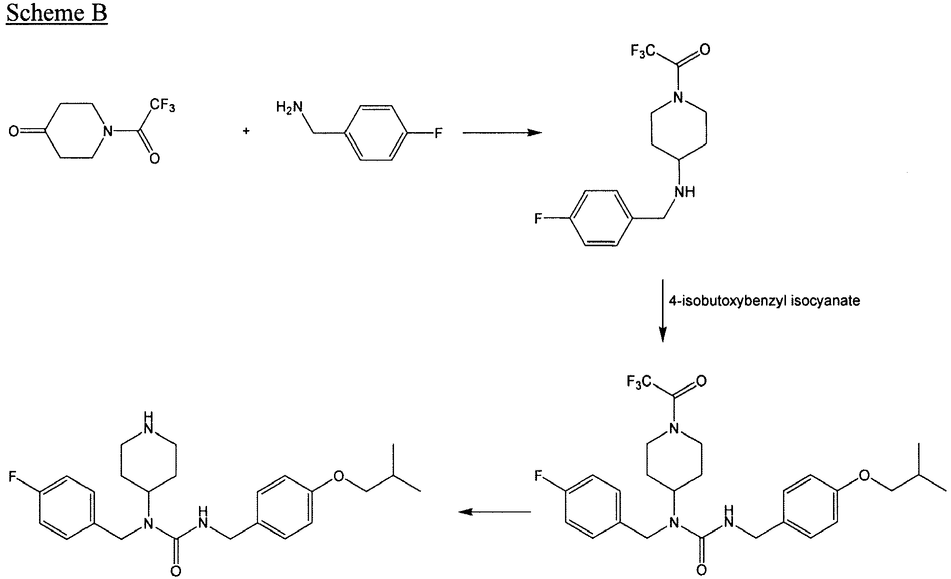

An exemplary method for synthesizing the compound of Formula (III) is shown in Scheme B. The protected 4-piperidoinone and 4-fluorobenzylamine can undergo reductive amination to form *N*-(4-fluorobenzyl)-4-amino-1-trifluoroacetylpiperidine. The resulting secondary amine can then be reacted with the appropriate isocyanate to form the nitrogen-protected carbamide. The acyl protecting group can be cleaved off using an alkali metal salt such as potassium carbonate to form the compound of Formula (III).

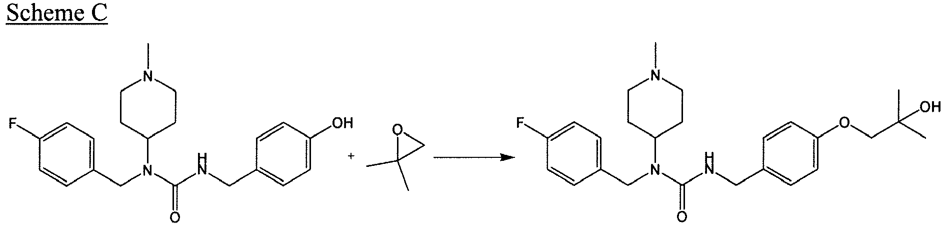

One method for synthesizing the compound of Formula (IV) is shown in Scheme C. The compound of Formula (II) can be reacted with isobutylene oxide to form the compound of Formula (IV) via a nucleophilic ring opening of the epoxide.

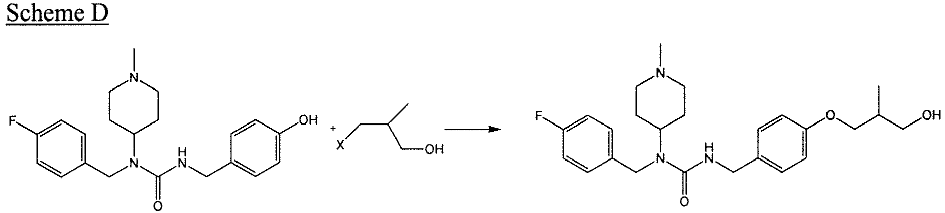

Scheme D shows a general reaction scheme for forming the compound of Formula (V). As shown in Scheme D, the compound of Formula (II) can be reacted with a halohydrin to form the compound of Formula (V). All the compounds described herein can be purified using methods known to those skilled in art.

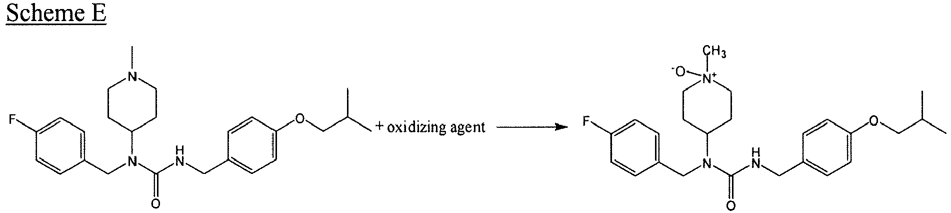

One example of a method for synthesizing a compound of Formula (VI) is shown in Scheme E. As shown in Scheme E, N-(1-methylpiperidin-4-yl)-N-(4-fluorophenylmethyl)-N'-(4-(2-methylpropyloxy)phenylmethyl) carbamide can be oxidized with a suitable oxidizing agent to form a compound of Formula (VI). Suitable oxidizing agents are known to those skilled in the art. One example of a suitable oxidizing agent is meta-chloroperbenzoic acid. All the compounds described herein can be purified using methods known to those skilled in art.

When pimavanserin is used herein, it is understood that salts, hydrates, polymorphs and isolated, substantially pure metabolites thereof, either individually or in combination could be used in place of pimavanserin. In an embodiment, the form of pimavanserin that can be used is its tartrate salt.

Some embodiments disclosed herein relate to the co-administration of pimavanserin with an agent that ameliorates one or more cholinergic abnormalities. By "co-administration," or, administration in "combination," it is meant that the two or more agents may be found in the subject's bloodstream at the same time, regardless of when or how they are actually administered. In some embodiments, pimavanserin can be administered at approximately the same time with the agent ameliorates one or more cholinergic abnormalities. As used herein, at approximately the same time means at substantially the same time or without a measure amount of time between. For example, simultaneous administration can be accomplished by combining pimavanserin and the agent that ameliorates one or more cholinergic abnormalities in a single dosage form. In another embodiment, pimavanserin and the agent that ameliorates one or more cholinergic abnormalities can be administered sequentially. For example, pimavanserin and the agent that ameliorates one or more cholinergic abnormalities can each be formulated as a separate dose. In an embodiment, pimavanserin can be administered before the agent that ameliorates one or more cholinergic abnormalities. In another embodiment, pimavanserin can be administered after the agent that ameliorates one or more cholinergic abnormalities. In another embodiment, a measurable amount of time elapses between each administration. In one embodiment, pimavanserin and the agent that ameliorates one or more cholinergic abnormalities can be administered through the same route, such as orally. In another embodiment, pimavanserin and the agent that ameliorates one or more cholinergic abnormalities are administered through different routes, such as one being administered orally and another being administered intravenously (i.v.). In one embodiment, the pharmacokinetics of pimavanserin and the agent that ameliorates one or more cholinergic abnormalities are substantially the same.

It has been surprisingly discovered that co-administration of pimavanserin with an agent that ameliorates one or more cholinergic abnormalities can synergistically affect the efficacy of the agent. Some embodiments disclosed herein relate to a composition that can include pimavanserin and an agent that ameliorates one or more cholinergic abnormalities. Some embodiments disclosed herein relate to a method for treating or ameliorating a disease condition by administering pimavanserin in combination with an agent that ameliorates one or more cholinergic abnormalities to attain a synergistic effect. In an embodiment, the agent can promote the activity of an M₁ muscarinic receptor.

As previously mentioned, administration of pimavanserin in combination with an agent that ameliorates one or more cholinergic abnormalities has been found to synergistically enhance the efficacy of the agent. Agents that ameliorate cholinergic abnormalities may act through a variety of mechanisms, including but not limited to, increasing acetylcholine concentration, increasing the activity of the muscarinic receptors (e.g., M1 receptor), rectifying alternations in choline transport, rectifying impaired acetylcholine release, addressing deficits in the nicotinic and muscarinic receptor expression, rectifying dysfunctional neurotrophin support, and/or rectifying deficits in axonal support. In some embodiments, the agent that ameliorates one or more cholinergic abnormalities can be selected from a cholinesterase inhibitor, a muscarinic agonist, a glutamatergic antagonist, a cholinergic agonist, a carnitine acetyltransferase stimulant, an acetylcholine release stimulant, and a choline uptake stimulant.

In some embodiments, the agent that ameliorates one or more cholinergic abnormalities can be a cholinesterase inhibitor. In an embodiment, the cholinesterase inhibitor can be selected from an acetylcholinesterase inhibitor and a butyrylcholinesterase inhibitor. Exemplary cholinesterase inhibitors include, but are not limited to, organophosphates such as metrifonate, echothiophate and diisopropyl fluorophosphate; carbamates such as physostigmine, neostigmine, pyridostigmine, ambenonium, demarcarium, rivastigmine, aldicarb, bendiocarb, bufencarb, carbaryl, carbendazim, carbetamide, carbofuran, chlorbufam, chloropropham, ethiofencarb, formetanate, methiocarb, methomyl, oxamyl, phenmedipham, pinmicarb, pirimicarb, propamocarb, propham, and propoxur; phenanthrine derivatives such as galantamine; piperidines such as donepezil; tacrine; edrophonium; phenothiazines;, dimebon (3, 6-dimethyl-9-(2-methyl-pyridyl-5)-ethyl-1,2,3,4-tetrahydro-γ-carboline dihydrochloride); Huperzine A; T-82 ((2-[2-(1-benzylpiperidin-4-yl)ethyl]-2,3-dihydro-9-methoxy-1H-pyrrolo[3,4-b]quinolin-1-one hemifumarate)); TAK-147 (zanapezil); phenserine; quilostigmine; ganstigmine; and butylcholinesterase inhibitors such as butyrophenones, imipramines, tropates, phencyclidines, curariforms, ethephon, ethopropazine, iso-OMPA, tetrahydrofurobenzofuran cymserine, N'phenethyl-norcymserine, N⁸-benzylnorcymserine, N¹, N⁸-bisnorcymserine , N¹-N⁸-bisbenzylnorphysostigmine , N¹, N⁸-bisbenzylnorphenserine and N¹, N⁸-bisbenzylnorcymserine.

In other embodiments, the agent that ameliorates one or more cholinergic abnormalities can be a muscarinic agonist. Exemplary muscarinic receptor agonists include, but are not limited to, xanomeline, carbamylcholine, oxotremorine, methacholine, bethanechol, cevimeline (AF102B), AF150(S), AF267B, aceclidine, arecoline, milameline, talsaclidine, pilocarpine and (S)-2-ethyl-8-methyl-1-thia-4,8-diaza-spiro[4.5]decan-3-one (Torrey Pines NGX267). In one embodiment, the muscarinic agonist can be xanomeline.

In still other embodiments, the agent that ameliorates one or more cholinergic abnormalities can be a glutamatergic antagonist. Exemplary glutamatergic antagonists include, but are not limited to, memantine, amantadine, dextromethorphan, dextrorphan, ibogaine, ketamine, tramadol, and methadone.

In some embodiments, the agent that ameliorates one or more cholinergic abnormalities can be a cholinergic agonist. Exemplary cholinergic agonists include, but are not limited to, pramiracetam, piracetam, oxiracetam, choline-L-alfoscerate, nebracetam, besipirdine, and taltirelin.

In other embodiments, the agent that ameliorates one or more cholinergic abnormalities can be a carnitine acetyltransferase stimulant. Exemplary carnitine acetyltransferase stimulants include, but are not limited to, levocarnitine, ST-200 (acetyl-1-carnitine), and nefiracetam.

In still other embodiments, the agent that ameliorates one or more cholinergic abnormalities can be an acetylcholine release stimulant. Exemplary acetylcholine release stimulants include, but are not limited to, SIB-1553A ((+/-)-4-[[2-(1-methyl-2-pyrrolidinyl)ethyl]thio]phenol hydrochloride) and T-588 ((1*R*)-1-benzo [*b*] thiophen-5-yl-2-[2-(diethylamino) ethoxy] ethan-1-ol hydrochloride).

In even other embodiments, the agent that ameliorates one or more cholinergic abnormalities can be a choline uptake stimulant. In an embodiment, the choline uptake stimulant can be MKC-231 (2-(2-oxopyrrolidin-1-yl)-N-(2,3-dimethyl-5,6,7,8-tetrahydrofuro [2,3-b]quinolin-4-yl)acetoamide).

Other embodiments disclosed herein relate to a pharmaceutical composition that can include pimavanserin, an agent that ameliorates one or more cholinergic abnormalities, and a pharmaceutically acceptable salt, carrier, diluent, and/or excipient.

As used herein, "pharmaceutically acceptable salt" refers to a salt of a compound that does not abrogate the biological activity and properties of the compound. Pharmaceutical salts can be obtained by reaction of a compound disclosed herein with an acid or base. Base-formed salts include, without limitation, ammonium salt (NH₄⁺); alkali metal, such as, without limitation, sodium or potassium, salts; alkaline earth, such as, without limitation, calcium or magnesium, salts; salts of organic bases such as, without limitation, dicyclohexylamine, N-methyl-D-glucamine, tris(hydroxymethyl)methylamine; and salts with the amino group of amino acids such as, without limitation, arginine and lysine. Useful acid-based salts include, without limitation, hydrochlorides, hydrobromides, sulfates, nitrates, phosphates, methanesulfonates, ethanesulfonates, p-toluenesulfonates and salicylates.

As used herein, a "carrier" refers to a compound that facilitates the incorporation of a compound into cells or tissues. For example, without limitation, dimethyl sulfoxide (DMSO) is a commonly utilized carrier that facilitates the uptake of many organic compounds into cells or tissues of a subject.

As used herein, a "diluent" refers to an ingredient in a composition that lacks pharmacological activity but may be pharmaceutically necessary or desirable. For example, a diluent may be used to increase the bulk of a potent drug whose mass is too small for manufacture or administration. It may also be a liquid for the dissolution of a drug to be administered by injection, ingestion or inhalation. A common form of diluent in the art is a buffered aqueous solution such as, without limitation, phosphate buffered saline that mimics the composition of human blood.

As used herein, an "excipient" refers to an inert substance that is added to a composition to provide, without limitation, bulk, consistency, stability, binding ability, lubrication, disintegrating ability etc., to the composition. A "diluent" is a type of excipient.

The pharmaceutical compositions disclosed herein may be manufactured in a manner that is itself known, *e.g*., by means of conventional mixing, dissolving, granulating, dragee-making, levigating, emulsifying, encapsulating, entrapping or tableting processes. Proper formulation is dependent upon the route of administration chosen. Techniques for formulation of the compositions described herein are known to those skilled in the art. The active ingredients are contained in an amount effective to achieve its intended purpose. Many of the compounds used in the pharmaceutical combinations disclosed herein may be provided as salts with pharmaceutically compatible counterions.

Some embodiments disclosed herein relate to a method for ameliorating or treating a disease condition characterized by one or more cholinergic abnormalities that can include administering a therapeutically effective amount of pimavanserin, and a therapeutically effective amount an agent that ameliorates one or more cholinergic abnormalities to a subject suffering from the disease condition characterized by one or more cholinergic abnormalities. Cholinergic abnormalities include, but are not limited to the following: alternations in choline transport, impaired acetylcholine release, deficits in the expression of nicotinic and muscarinic receptors, dysfunctional neurotrophin support and deficits in axonal transport. The aforementioned are classified as abnormalities when compared to a healthy subject. Other embodiments disclosed herein relate to a method for ameliorating or treating a disease condition characterized by one or more cholinergic abnormalities that can include administering a therapeutically effective amount of pimavanserin, and a therapeutically effective amount of an agent that ameliorates one or more cholinergic abnormalities to a subject suffering from the disease condition characterized by one or more cholinergic abnormalities. In some embodiments, the agent that ameliorates one or more cholinergic abnormalities can be selected from a cholinesterase inhibitor, a muscarinic agonist, and a glutamatergic agonist. In an embodiment, the agent promotes the activity of an M₁ muscarinic receptor.

Conditions suitable for treatment by a composition disclosed herein include, but are not limited to, cognitive impairment, forgetfulness, confusion, memory loss, an attention deficit disorder, deficits in visual perception, depression, pain, sleep disorders, psychosis, increased intraocular pressure, neurodegenerative diseases, Alzheimer's disease, Parkinson's disease, schizophrenia, Huntington's chorea, Friederich's ataxia, Gilles de la Tourette's Syndrome, Down Syndrome, Pick disease, dementia, clinical depression, age-related cognitive decline, attention-deficit disorder, sudden infant death syndrome, glaucoma, mania, bipolar disorder, unipolar disorder, schizoaffective disorder, schizophreniform disorder and anxiety. It should be noted that other non-schizophrenic causes of psychosis, including drug-induced and treatment-induced, those associated with dementia and other neurodegenerative disorders (such as Huntington's and Alzheimer's) are also suitable.

In some embodiments, pimavanserin and an agent that ameliorates one or more cholinergic abnormalities can be administered in combination to treat, ameliorate, or prevent a neuropsychiatric disorder, including but not limited to schizophrenia, schizoaffective disorders, mania, depression (including dysthymia, treatment-resistant depression, and depression associated with psychosis), cognitive disorders, aggressiveness (including impulsive aggression), a panic attack, an obsessive compulsive disorder, borderline personality disorder, borderline disorder, multiplex developmental disorder (MDD), a behavioral disorder (including behavioral disorders associated with age-related dementia), psychosis (including psychosis associated with dementia, induced by treatment, such as treatment of Parkinson's disease, or associated with post traumatic stress disorder), suicidal tendency, bipolar disorder, sleep disorder (including sleep maintenance insomnia, chronic insomnia, transient insomnia, and periodic limb movements during sleep (PLMS)), addiction (including drug or alcohol addiction, opioid addiction, and nicotine addiction), attention deficit hyperactivity disorder (ADHD), post traumatic stress disorder (PTSD), Tourette's syndrome, anxiety (including general anxiety disorder (GAD)), autism, Down's syndrome, a learning disorder, a psychosomatic disorder, alcohol withdrawal, epilepsy, pain (including chronic pain, neuropathic pain, inflammatory pain, diabetic peripheral neuropathy, fibromyalgia, postherpetic neuralgia, and reflex sympathetic dystrophy), a disorder associated with hypoglutamatergia (including schizophrenia, childhood autism, and dementia), and serotonin syndrome.

In some embodiments, pimavanserin can be administered in combination with an agent that ameliorates one or more cholinergic abnormalities to treat, ameliorate, or prevent a neurodegenerative disorder. Exemplary neurodegenerative disorders include but are not limited to, Alzheimer's disease (including psychosis and/or dementia associated with Alzheimer's disease), Parkinson's disease, Huntington's chorea, sphinocerebellar atrophy, frontotemporal dementia, supranuclear palsy, or Lewy body dementia. In one embodiment, the co-administration of pimavanserin and an agent that ameliorates one or more cholinergic abnormalities can be used to treat Alzheimer's disease with psychosis. In some embodiments, symptoms associated with Alzheimer's disease that can be treated, include, but are not limited to, agitation, aggression, delusions, hallucinations, depression, insomnia, and anxiety.

In some embodiments, pimavanserin can be administered in combination with an agent that ameliorates one or more cholinergic abnormalities to treat, ameliorate, or prevent an extrapyramidal disorder. Examples of extrapyramidal disorders include, but are not limited to, dyskinesias (such as induced by treatment of Parkinson's disease), bradykinesia, rigidity, psychomotor slowing, tics, akathisia (such as induced by a neuroleptic or SSRI agent), Friedrich's ataxia, Machado-Joseph's disease, dystonia, tremor, restless legs syndrome, and myoclonus.

In some embodiments, pimavanserin can be administered in combination with an agent that ameliorates one or more cholinergic abnormalities to treat, ameliorate, or prevent psychosis. Functional causes of the psychosis may include schizophrenia; a bipolar disorder; severe clinical depression; severe psychosocial stress; sleep deprivation; a neurological disorder including a brain tumor; dementia with Lewy bodies; multiple sclerosis; sarcoidosis; an electrolyte disorder including hypocalcemia, hypernatremia, hyonatremia, hyopkalemia, hypomagnesemia, hypermagnesemia, hypercalcemia, hypophosphatemia, and hypoglycemia; lupus; AIDS; leprosy; malaria; flu; mumps; psychoactive drug intoxication; withdrawal including alcohol, barbiturates, benzodiazepines, anticholinergics, atropine, scopolamine, Jimson weed, antihistamines, cocaine, amphetamines, and hallucinogens such as cannabis, LSD, psilocybin, mescaline, MDMA, and PCP. Psychosis may include symptoms such as delusions, hallucinations, disorganized speech, disorganized behavior, gross distortion of reality, impaired mental capacity, impaired affective response, fluctuating level of consciousness, poor motor co-ordination, inability to perform simple mental tasks, disorientation as to person, place or time, confusion, and/or memory impairment. In one embodiment, the subject can be experiencing acute exacerbation of psychosis. In some embodiments, a combination described herein can be used to treat schizophrenia. In an embodiment, the psychosis can be associated with schizophrenia. In one embodiment, the subject has exhibited a prior response, or is currently exhibiting a response, to antipsychotic therapy. In one embodiment, the subject exhibits a moderate degree of psychopathology.

In one embodiment, the co-administration of pimavanserin with an agent that ameliorates one or more cholinergic abnormalities can be used to treat, ameliorate, or prevent depression.

In some embodiments, pimavanserin potentiates the ability of an agent that ameliorates one or more cholinergic abnormalities to have its therapeutic effect. For example, in some embodiments, pimavanserin potentiates the ability of a cholinesterase inhibitor, a muscarinic agonist, and/or a glutamatergic antagonist to have its therapeutic effect. In some embodiments, pimavanserin potentiates the ability of a cholinesterase inhibitor, a muscarinic agonist, and/or a glutamatergic antagonist to treat psychosis and/or improve cognition.

In some embodiments, the co-administration of pimavanserin with an agent that ameliorates one or more cholinergic abnormalities can be used to treat, ameliorate or prevent chemotherapy-induced emesis, frailty, on/off phenomena, non-insulin-dependent diabetes mellitus, metabolic syndrome, an autoimmune disorder (including lupus and multiple sclerosis), sepsis, increased intraocular pressure, glaucoma, a retinal disease (including age related macular degeneration), Charles Bonnet syndrome, substance abuse, sleep apnea, pancreatis, anorexia, bulimia, a disorder associated with alcoholism, cerebral vascular accidents, amyotrophic lateral sclerosis, AIDS related dementia, traumatic brain or spinal injury, tinnitus, menopausal symptoms (such as hot flashes), sexual dysfunction (including female sexual dysfunction, female sexual arousal dysfunction, hypoactive sexual desire disorder, decreased libido, pain, aversion, female orgasmic disorder, and ejaculatory problems), low male fertility, low sperm motility, hair loss or thinning, incontinence, hemorrhoids, migraine, hypertension, thrombosis (including thrombosis associated with myocardial infarction, stroke, idiopathic thrombocytopenic purpura, thrombotic thrombocytopenic purpura, and peripheral vascular disease), abnormal hormonal activity (such as abnormal levels of ACTH, corticosterone, rennin, or prolactin), a hormonal disorder (including Cushing's disease, Addison's disease, and hyperprolactinemia), a pituitary tumor (including a prolactinoma), a side effect associated with a pituitary tumor (including hyperprolactinemia, infertility, changes in menstruation, amenorrhea, galactorrhea, loss of libido, vaginal dryness, osteoporosis, impotence, headache, blindness, and double vision), vasospasm, ischemia, a cardiac arrhythmia, cardiac insufficiency, asthma, emphysema, and/or an appetite disorder.

In some embodiments, pimavanserin can be used to treat, ameliorate and/or prevent psychosis. In an embodiment, the psychosis is Alzheimer's disease-induced psychosis.

As used herein, the terms "treating," "treatment," "therapeutic," or "therapy" do not necessarily mean total cure or abolition of the disease or condition. Any alleviation of any undesired signs or symptoms of a disease or condition, to any extent can be considered treatment and/or therapy. Furthermore, treatment may include acts that may worsen the subject's overall feeling of well-being or appearance.

The term "therapeutically effective amount" is used to indicate an amount of an active compound, or pharmaceutical agent, that elicits the biological or medicinal response indicated. For example, a therapeutically effective amount of compound can be the amount need to prevent, alleviate or ameliorate symptoms of disease or prolong the survival of the subject being treated This response may occur in a tissue, system, animal or human and includes alleviation of the symptoms of the disease being treated. Determination of a therapeutically effective amount is well within the capability of those skilled in the art, especially in light of the detailed disclosure provided herein. The therapeutically effective amount of the compounds disclosed herein required as a dose will depend on the route of administration, the type of animal, including human, being treated, and the physical characteristics of the specific animal under consideration. The dose can be tailored to achieve a desired effect, but will depend on such factors as weight, diet, concurrent medication and other factors which those skilled in the medical arts will recognize.

As used herein, a "subject" refers to an animal that is the object of treatment, observation or experiment. "Animal" includes cold- and warm-blooded vertebrates and invertebrates such as fish, shellfish, reptiles and, in particular, mammals. "Mammal" includes, without limitation, mice, rats, rabbits, guinea pigs, dogs, cats, sheep, goats, cows, horses, primates, such as monkeys, chimpanzees, and apes, and, in particular, humans.

The compositions described herein can be administered to a human subject *per se,* or in pharmaceutical compositions where they are mixed with other active ingredients, as in combination therapy, or carriers, diluents, excipients or combinations thereof. Techniques for administrating the compositions described herein are known to those skilled in the art. Suitable routes of administration may, for example, include oral, rectal, topical transmucosal, or intestinal administration; parenteral delivery, including intramuscular, subcutaneous, intravenous, intramedullary injections, as well as intrathecal, direct intraventricular, intraperitoneal, intranasal, intraocular injections or as an aerosol inhalant.

One may also administer the composition in a local rather than systemic manner, for example, via injection of the compound directly into the infected area, often in a depot or sustained release formulation.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, may be the labeling approved by the U.S. Food and Drug Administration for prescription drugs, or the approved product insert. Compositions that can include a compound described herein formulated in a compatible pharmaceutical carrier may also be prepared, placed in an appropriate container, and labeled for treatment of an indicated condition.

As will be readily apparent to one skilled in the art, the useful *in vivo* dosage to be administered and the particular mode of administration will vary depending upon the age, weight, the severity of the affliction, and mammalian species treated, the particular compounds employed, and the specific use for which these compounds are employed. (See *e.g.,* Fingl *et al.* 1975, in "The Pharmacological Basis of Therapeutics", with particular reference to Ch. 1, p. 1). The determination of effective dosage levels, that is the dosage levels necessary to achieve the desired result, can be accomplished by one skilled in the art using routine pharmacological methods. Typically, human clinical applications of products are commenced at lower dosage levels, with dosage level being increased until the desired effect is achieved. Alternatively, acceptable *in vitro* studies can be used to establish useful doses and routes of administration of the compositions identified by the present methods using established pharmacological methods. In cases of administration of a pharmaceutically acceptable salt, dosages may be calculated as the free base.

Although the exact dosage will be determined on a drug-by-drug basis, in most cases, some generalizations regarding the dosage can be made. Typically, the dose range of the composition administered to the subject can be from about 0.5 to about 1000 mg/kg of the subject's body weight, or about 1 to about 500 mg/kg, or about 10 to about 500 mg/kg, or about 50 to about 100 mg/kg of the subject's body weight. Additionally, the daily dosage regimen for an adult human subject may be, for example, an oral dose of between about 0.1 mg and about 500 mg of each ingredient, preferably between about 1 mg and about 250 mg, e.g. about 5 to about 200 mg or an intravenous, subcutaneous, or intramuscular dose of each ingredient between about 0.01 mg and about 100 mg, preferably between about 0.1 mg and about 60 mg, e.g. about 1 to about 40 mg of each ingredient of the pharmaceutical compositions disclosed herein or a pharmaceutically acceptable salt thereof calculated as the free base. The dosage may be a single one or a series of two or more given in the course of one or more days, as is needed by the subject. In some embodiments, the compounds will be administered for a period of continuous therapy, for example for a week or more, or for months or years.

In instances where human dosages for compounds have been established for at least some condition, the composition will use those same dosages, or dosages that are between about 0.1% and about 500%, more preferably between about 25% and about 250% of the established human dosage. Where no human dosage is established, as will be the case for newly-discovered pharmaceutical compositions, a suitable human dosage can be inferred from ED₅₀ or ID₅₀ values, or other appropriate values derived from *in vitro* or *in vivo* studies, as qualified by toxicity studies and efficacy studies in animals.

As will be understood by those of skill in the art, in certain situations it may be necessary to administer the compounds disclosed herein in amounts that exceed, or even far exceed, the above-stated, preferred dosage range in order to effectively and aggressively treat particularly aggressive diseases or infections.

Dosage amount and interval may be adjusted individually to provide plasma levels of the active moiety which are sufficient to maintain the modulating effects, or minimal effective concentration (MEC). The MEC will vary for each compound but can be estimated from in vitro data. Dosages necessary to achieve the MEC will depend on individual characteristics and route of administration. However, HPLC assays or bioassays can be used to determine plasma concentrations.

Dosage intervals can also be determined using MEC value. Compositions should be administered using a regimen which maintains plasma levels above the MEC for 10-90% of the time, preferably between 30-90% and most preferably between 50-90%. In cases of local administration or selective uptake, the effective local concentration of the drug may not be related to plasma concentration.

It should be noted that the attending physician would know how to and when to terminate, interrupt, or adjust administration due to toxicity or organ dysfunctions. Conversely, the attending physician would also know to adjust treatment to higher levels if the clinical response were not adequate (precluding toxicity). The magnitude of an administrated dose in the management of the disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual subject. A program comparable to that discussed above may be used in veterinary medicine.

In non-human animal studies, applications of potential products are commenced at higher dosage levels, with dosage being decreased until the desired effect is no longer achieved or adverse side effects disappear. The dosage may range broadly, depending upon the desired effects and the therapeutic indication. Alternatively dosages may be based and calculated upon the surface area of the subject, as understood by those of skill in the art.

Compounds disclosed herein can be evaluated for efficacy and toxicity using known methods. For example, the toxicology of a particular compound, or of a subset of the compounds, sharing certain chemical moieties, may be established by determining *in vitro* toxicity towards a cell line, such as a mammalian, and preferably human, cell line. The results of such studies are often predictive of toxicity in animals, such as mammals, or more specifically, humans. Alternatively, the toxicity of particular compounds in an animal model, such as mice, rats, rabbits, or monkeys, may be determined using known methods. The efficacy of a particular compound may be established using several recognized methods, such as *in vitro* methods, animal models, or human clinical trials. Recognized *in vitro* models exist for nearly every class of condition, including but not limited to cancer, cardiovascular disease, and various immune dysfunction. Similarly, acceptable animal models may be used to establish efficacy of chemicals to treat such conditions. When selecting a model to determine efficacy, the skilled artisan can be guided by the state of the art to choose an appropriate model, dose, and route of administration, and regime. Of course, human clinical trials can also be used to determine the efficacy of a compound in humans.

### EXAMPLES

**Chemistry.** ¹H NMR spectra were recorded at 400 MHz on a Varian Mercury-VX400MHz spectrometer and chemical shifts are given in δ-values [ppm] referenced to the residual solvent peak chloroform (CDCl₃) at 7.26 and methanol (CD₃OD) at 3.31 ppm. Coupling constants, *J*, are reported in Hertz. Unless otherwise stated, the NMR spectra of the compounds are described for their free amine form. Column chromatography was carried out using silica gel 60 (particle size 0.030-0.070 mm) from Merck. Materials and solvents were of the highest grade available from commercial sources and used without further purification. Reversed phase C₁₈ solid phase extraction cartridges (SPE) were DSC-18 2 g/12 mL columns from Discovery™ Solid Phase Extraction Products, Supelco. Preparative HPLC was run on a Waters/Micromass HPLC/MS using a diode array detector (190-450 nm) UV detector and Micromass ZMD-mass-spectrometer with electrospray ionization. A YMC J'sphere ODS H80 19x100 mm column was used. The mobile phase was 0.15% TFA in water/acetonitrile with a gradient starting at 30% acetonitrile, going to 100% acetonitrile over 13 min. The flow rate was 17 mL/min.

**HPLC/LCMS Method.** Samples were run on a Waters/Micromass HPLC/MS using a diode array detector (190-450 nm) UV detector and Micromass ZMD-mass-spectrometer with electrospray ionization. A Phenomenex Luna C₁₈(2) 3 µm, 75 × 4.6 mm column was used. The mobile phase was 10 mM ammonium acetate in water/acetonitrile with a gradient starting at 30% acetonitrile, going to 95% acetonitrile over 12 min. The flow rate was 1.0 mL/min.

**Preparation of hydrochloride salts.** The tertiary amine products were dissolved in dichloromethane, treated with an excess of 1M HCl in diethyl ether and precipitated from n-heptane. The solvents were removed *in vacuo* and after drying, the hydrochloride salts were obtained as colorless solids in quantitative yield.

### N-(4-Fluorobenzyl)-N-(1-methylpiperidin-4-yl)-N'-(4-hydroxybenzyl)carbamide hydrochloride

*N*-((4-Fluorophenyl)methyl)-4-amino-1-methylpiperidine was prepared from 1-methylpiperidine-4-one (1.15 mL, 10 mmol), which was dissolved in methanol (30 mL). 4-Fluorobenzylamine (1.25 mL, 10 mmol) was added and the pH was adjusted to 5 with acetic acid. NaBH₃CN (1.25 g, 20 mmol) was added and the reaction mixture was stirred for 3h, after which it was concentrated. 2M aqueous NaOH (30 mL) was added and the mixture extracted with dichloromethane (2x50 mL). The combined organic phases were dried over Na₂SO₄, filtered and evaporated, and this crude product was purified by Kugelrohr distillation to give the desired product (1.1 g, 50%) as a clear oil.

*N*-(4-Fluorobenzyl)-4-amino-1-methylpiperidine (4.00 g, 18.0 mmol) was dissolved in dichloromethane (150 mL). 4-Methoxybenzyl isocyanate (3.26 g, 20.0 mmol) in dichloromethane (50 mL) was added dropwise and the mixture was stirred for 3h at room temperature. The crude mixture was concentrated and purified by flash chromatography (0-10% methanol in dichloromethane) to give *N*-((4-fluorophenyl)methyl)-*N*-(1-methylpiperidin-4-yl)-*N*'-((4-methoxyphenyl)methyl)carbamide (4.91 g, 71%). This carbamide (4.91 g, 13.0 mmol) was dissolved in dry dichloromethane (50 mL). The solution was cooled to 0°C and boron tribromide (1M in dichloromethane, 39.0 mL, 39.0 mmol) was added dropwise, and the mixture stirred for 20 h at room temperature. Water (50 mL) and n-butanol (10 mL) were added and the phases separated. The aqueous phase was extracted a second time with a mixture of dichloromethane (50 mL) and n-butanol (10 mL). The combined organic phases were evaporated and the resulting solid was purified by flash chromatography (0-20% methanol in dichloromethane) to give a semi-pure solid (3.17 g, 67%). An analytical amount (25 mg) of this material was purified by preparative HPLC to give a colorless oil (10 mg). LC-MS showed [M+H]⁺=372 (characteristic fragment: 223). ¹H-NMR (CD₃OD, 400 MHz, Free base): δ 7.25-6.62 (m, 8H), 4.46 (s, 2H), 4.22 (s, 2H), 4.15-4.06 (m, 1H), 2.89-2.82 (m, 2H), 2.23 (s, 3H), 2.14-2.05 (m, 2H), 1.74-1.61 (m, 4H).

The collected compound was converted into its hydrochloride salt, which was obtained as a colorless solid.

### N-(4-Fluorobenzyl)-N-(piperidin-4-yl)-N'-(4-isobutoxybenzyl)carbamide hydrochloride

4-Piperidone hydrochloride monohydrate (4.0 g, 26.0 mmol) was dissolved in dichloromethane (130 mL). After addition of triethylamine (8.66 g, 85.8 mmol) the mixture was stirred for 10 min and then cooled to 0°C. Trifluoroacetic anhydride (12.0 g, 57.2 mmol) was added dropwise under stirring. After 2 hours at room temperature, the reaction was stopped by addition of water (100 mL). The aqueous phase was extracted with dichloromethane (2x100 mL). The combined organic phases were dried over Na₂SO₄, filtered and concentrated to give 1-trifluoroacetyl-4-piperidone (5.07 g, 100%). 4-Fluorobenzylamine (3.14 g, 25.9 mmol) and 1-trifluoroacetyl-4-piperidone (5.07 g, 25.9 mmol) were added to a solution of methanol adjusted to pH 5 with acetic acid (150 mL). The reaction mixture was stirred for 5 min and NaBH₃CN (2.46 g, 38.9 mmol) was added slowly under stirring. After 20 hours at room temperature the reaction was concentrated. 2M aqueous NaOH (100 mL) was added and extracted with dichloromethane (2x100 mL). The combined organic phases were dried over Na₂SO₄, filtered and concentrated to give N-(4-fluorobenzyl)-4-amino-1-trifluoroacetylpiperidine (2.91 g, 37%).

4-Isobutoxybenzyl isocyanate was prepared from 4-isobutoxyphenylacetic acid (7.6 g, 36.5 mmol) (prepared according to classical literature procedures from methyl 4-hydroxyphenylacetate by a Williamson ether synthesis with isobutylbromide, followed by saponification of the ester. For an alternative route see: Profft; Drux; J. Prakt. Chem. 1956, 4(3), 274-275), and which was dissolved in THF (50 mL). Proton Sponge™ (8.2 g, 38 mmol) was added, and the mixture was stirred for 15 min. Diphenylphosphoryl azide (10.6 g, 38 mmol) was added dropwise and the mixture was heated to reflux for 4h. The mixture was cooled to room temperature and placed in the freezer at -18°C for 20h. The resulting white precipitate was vigorously stirred with diethyl ether (250 mL) for 15 min and filtered. The filtrate was evaporated to give the desired product, which was used without further purification.

*N*-(4-fluorobenzyl)-4-amino-1-trifluoroacetylpiperidine (2.91 g, 9.6 mmol) was dissolved in dichloromethane (50 mL) and a solution of 4-isobutoxybenzyl isocyanate (1.97 g, 9.6 mmol) in dichloromethane (50 mL) was added. The reaction mixture was stirred for 20h and concentrated. The crude product was purified by flash chromatography (0-5% methanol in dichloromethane) to give *N*-(4-fluorobenzyl)-*N*-(1-trifluoroacetylpiperidin-4-yl)-*N*'-(4-isobutoxybenzyl)carbamide (3.90 g, 91%).

This carbamide (3.90 g, 8.7 mmol) was dissolved in methanol (12 mL) and added to a 2M solution of potassium carbonate in methanol (100 mL) under stirring. After 4 hours the methanol was evaporated, and the aqueous phase was extracted with dichloromethane (2x100 mL). The combined organic phases were dried over Na₂SO₄, filtered and concentrated to give a semi-pure solid (2.95 g, 85%). An analytical amount (200 mg) of this crude product was purified by flash chromatography (10% methanol in dichloromethane with 1% triethylamine) to give a colorless solid (100 mg). LC-MS showed [M+H]⁺=414 (characteristic fragment: 209). ¹H-NMR (CDCl₃, 400 MHz, Free base): δ 7.21-6.75 (m, 8H), 4.47-4.42 (m, 1H), 4.39 (t, *J*=5.0 Hz, 1H), 4.35 (s, 2H), 4.27 (d, *J*=5.0 Hz, 2H), 3.68 (d, *J*=6.0 Hz, 2H), 3.13-3.06 (m, 2H), 2.74-2.66 (m, 2H), 2.11-1.99 (m, 1H), 1.78-1.71 (m, 3H), 1.58-1.46 (m, 2H), 1.00 (d, *J*=6.0 Hz, 6H).

The collected compound was converted into its hydrochloride salt, which was obtained as a colorless solid.

### N-4-Fluorobenzyl)-N-(1-methylpiperidin-4-yl)-N'-14-(2-hydroxy)isobutoxybenzyl] carbamide hydrochloride

*N*-(4-Fluorobenzyl)-*N*-(1-methylpiperidin-4-yl)-*N*'-(4-hydroxybenzyl) carbamide (375 mg, 1.0 mmol) was dissolved in DMF (15 mL). KOH (281 mg, 5.0 mmol) was added and the mixture was stirred 30 min at room temperature. Isobutylene oxide (216 mg, 3.0 mmol) was added and the mixture was warmed to 40°C for 20h. Isobutylene oxide (216 mg, 3.0 mmol) was added and the mixture was stirred at 40°C for another 20h. Water (50 mL) was added and the mixture was extracted with dichloromethane (2x60 mL). The combined organic phases were dried over Na₂SO₄, filtered and evaporated. The crude product was purified by flash chromatography (5% methanol in dichloromethane) and subsequently by passage over a C₁₈-SPE cartridge, eluting with 30% acetonitrile/water and 3.5 mM ammonium acetate buffer. The acetonitrile was evaporated and the water phase was made alkaline with aqueous ammonia. The product was extracted into dichloromethane (2x100 mL), and the combined organic phases were dried over Na₂SO₄, filtered and evaporated to give a colorless oil (122 mg, 28%). LC-MS showed [M+H]⁺=444 (characteristic fragment: 223). ¹H-NMR (CDCl₃, 400 MHz, Free base): δ 7.21-6.77 (m, 8H), 4.49-4.43 (t, *J*=*5.5* Hz, 1H), 4.37-4.26 (m, 5H), 3.75 (s, 2H), 2.89-2.82 (m, 2H), 2.25 (s, 3H), 2.10-2.01 (m, 2H), 1.76-1.58 (m, 4H), 1.33 (s, 6H).

The collected compound was converted into its hydrochloride salt, which was obtained as a colorless solid.

### N-(4-Fluorobenzyl)-N-(1-methyl-piperidin-4-yl)-N'-(4-(R)-[(3-hydroxy)-isobutoxy]benzyl)carbamide

*N*-(4-Fluorobenzyl)-*N*-(1-methylpiperidin-4-yl)-*N*-(4-hydroxybenzyl)carbamide (75 mg, 0.20 mmol) was dissolved in DMF (3 mL). Potassium hydroxide (56 mg, 1.00 mmol) was added and the mixture was stirred 15 minutes at room temperature. (R)-(-)-3-Bromo-2-methyl-1-propanol (93 mg, 0.60 mmol) was added. The mixture was heated to 60°C for 5 hours. The reaction mixture was cooled to room temperature, added to dichloromethane (50 mL), and washed with 1M potassium hydroxide (50 mL). The organic phase was dried over Na₂SO₄, filtered and evaporated. The resulting oil was purified by preparative HPLC to give *N*-(4-Fluorobenzyl)-*N*-(1-methyl-piperidin-4-yl)-*N-*-(4-(R)-[(3-hydroxy)-isobutoxy]benzyl)carbamide as a colorless oil (5 mg, 6%). LC-MS showed [M+H]⁺ = 444 (characteristic fragment: 223). ¹H-NMR (CDCl₃, 400 MHz, free base): δ 7.20-6.78 (m, 8H), 4.47 (t, *J* = 5 Hz, 1H), 4.35-4.29 (m, 3H), 4.27 (d, *J* = 5.0 Hz, 2H), 3.92-3.89 (m, 2H), 3.68 (d, J = 6.0 Hz, 2H), 2.89-2.82 (m, 2H), 2.25 (s, 3H), 2.22-2.13 (m, 1H), 2.10-2.02 (m, 2H), 1.83-1.76(bs, 1H), 1.75-1.59 (m, 4H) 1.02 (d, *J* = 6.0 Hz, 3H).

The collected compound was converted into its hydrochloride salt, which was obtained as a colorless solid.

### N-(4-Fluorobenzyl)-N-(1-methyl-1-oxopiperidin-4-yl)-N'-(4-isobutoxybenzyl)carbamide

*N*-(4-Fluorobenzyl)-*N*-(1-methylpiperidin-4-yl)-*N*'-(4-isobutoxybenzyl)carbamide (100 mg, 0.234 mmol) was dissolved in dichloromethane (10 mL). The solution was cooled to 0°C and meta-chloroperbenzoic acid (57-86%, 106 mg, 0.351 mmol) was added. The reaction mixture was stirred for 20h at room temperature, after which it was washed with saturated aqueous NaHCO₃ (10 mL). The organic phase was dried over Na₂SO₄, filtered and evaporated. The resulting oil was purified by preparative HPLC to give *N*-(4-fluorobenzyl)-*N*-(1 -methyl-1 -oxopiperidin-4-yl)-*N*'-(4-isobutoxybenzyl)carbamide as a colorless oil (10 mg, 10%). LC-MS showed [M+H]⁺=444 (characteristic fragment: 239). ¹H-NMR (CDCl₃, 400 MHz): δ 7.20-6.76 (m, 8H), 4.63-4.53 (m, 2H), 4.43 (s, 2H), 4.24 (d, *J*=5.0 Hz, 2H), 3.66 (d, *J*=7.0 Hz, 2H), 3.31-3.24 (m, 4H), 3.19 (s, 3H), 2.62-2.51 (m, 2H), 2.10-1.99 (m, 1H), 1.69-1.62 (m, 2H), 1.00 (d, *J*=7.0 Hz, 6H).

### In vitro determination of receptor activity

**Receptor Selection and Amplification (R-SAT) Assays.** The functional receptor assay, Receptor Selection and Amplification Technology (R-SAT®), was used (with minor modifications from the procedure described previously (Brann, M. R. US Patent 5,707,798, 1998; *Chem. Abstr.* **1998,** *128,* 111548) to screen compounds for efficacy at the 5-HT_{2A} receptor. Briefly, NIH3T3 cells were grown in 96 well tissue culture plates to 70-80% confluence. Cells were transfected for 12-16 h with plasmid DNAs using superfect (Qiagen Inc.) as per manufacturer's protocols. R-SAT's were generally performed with 50 ng/well of receptor and 20 ng/well of β-galactosidase plasmid DNA. All receptor and G-protein constructs used were in the pSI mammalian expression vector (Promega Inc) as described previously. The 5-HT_{2A} or 5-HT_{2C} receptor gene was amplified by nested PCR from brain cDNA using the oligodeoxynucleotides based on the published sequence (Saltzman et. al, Biochem. Biophys. Res. Comm. 1991, 181, 1469). For large-scale transfections, cells were transfected for 12-16 h, then trypsinized and frozen in DMSO. Frozen cells were later thawed, plated at 10,000-40,000 cells per well of a 96 well plate that contained drug. With both methods, cells were then grown in a humidified atmosphere with 5% ambient CO₂ for five days. Media was then removed from the plates and marker gene activity was measured by the addition of the β-galactosidase substrate *o*-nitrophenyl β-D-galactopyranoside (ONPG, in PBS with 5% NP-40). The resulting colorimetric reaction was measured in a spectrophotometric plate reader (Titertek Inc.) at 420 nM. All data were analyzed using the computer program XLFit (IDBSm). Efficacy is the percent maximal repression compared to repression by a control compound (ritanserin in the case of 5-HT_{2A}). pIC₅₀ is the negative of the log(IC₅₀), where IC₅₀ is the calculated concentration in Molar that produces 50% maximal repression. Various metabolites of N-(1-methylpiperidin-4-yl)-N-(4-fluorophenylmethyl)-N'-(4-(2-methylpropyloxy)phenylmethyl) carbamide, including the compounds of Formulae (II), (III), (IV), (V) and (VI) as well as other metabolites, were assayed as described herein. The assayed metabolites demonstrated varying activity levels with some of the metabolites exhibiting levels too low for use as pharmaceuticals agents. Compounds of Formulae (II), (III), (IV), (V) and (VI), however, demonstrated high inverse agonist and antagonist activity as shown in the table below. This data indicates compounds of Formulae (II), (III), (IV), (V) and (VI) could be useful as pharmaceutical agents.

| **Compound** | **Inverse Agonist pIC₅₀** | | **Antagonist pKi** | |
|---|---|---|---|---|
| | **5HT2A** | **5HT2C** | **5HT2A** | **5HT2C** |
| Formula (II) | 7.5 | - | 7.9 | 6.5 |
| Formula (III) | 8.6 | - | 8.9 | 7 |
| Formula (IV) | 8.7 | 6.1 | 9 | 6.8 |
| Formula (V) | 8.5 | 6.7 | - | 6.8 |
| Formula (VI) | 7.5 | 5.7 | 7.5 | 6.4 |

### Example 1 - Amphetamine-Induced Hyperactivity with Tacrine

This study was conducted in acrylic chambers (42 cm x 42 cm x 30 cm) equipped with 16 infrared photobeams along each horizontal axis (front-to-back and side-to-side, from Accuscan Instruments, Inc., Columbus, OH). Mice were initially administered either vehicle (veh) or pimavanserin (0.03 mg/kg) 60 minutes prior to entering activity chambers. Vehicle or a drug (either tacrine or xanomeline) was injected 30 minutes prior to entering activity chambers. Amphetamine (3 mg/kg) was injected into mice 15 minutes prior to entering motor activity chambers. Mice had no prior exposure to the chambers and each dose combination was tested in separate groups of mice.

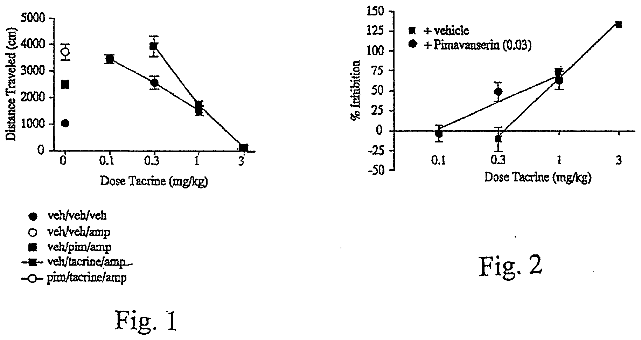

Activity was measured as total distance traveled (DT) in centimeters and was determined across a 15 minute session. Dose response curves were constructed for the drug in the presence of either vehicle or a fixed dose of pimavanserin. In order to generate dose-response curves, raw DT data were converted to percent inhibition, %MPI = ((DT drug or drug combination - DT amphetamine control)/(DT vehicle control - DT amphetamine control)) * 100. A linear regression analysis was then conducted to determine the theoretical dose that would produce a 50% reduction of activity (ED₅₀) and 95% confidence interval. The results of these studies for tacrine and xanomeline are presented in Figures 1-4.

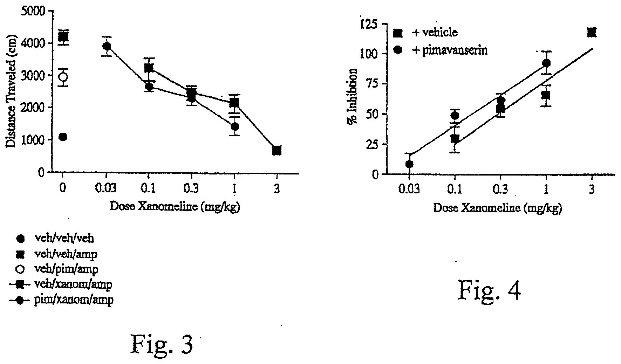

Figure 1 illustrates the total distance traveled as a function of the dosage of tacrine administered in mice given a vehicle control or amphetamine. As shown in Figure 1, as the dosage of tacrine was increased, the total distance traveled decreased. This shows the ability of tacrine to suppress amphetamine-induced hyperactivity in mice. Also shown in Figure 1 is that the administration of tacrine in combination with pimavanserin further reduced the total distance traveled of mice given amphetamine for a particular dosage of tacrine. Figure 2 illustrates the percent inhibition of amphetamine-induced hyperactivity in mice as a function of tacrine dosage. Increasing doses of tacrine resulted in a greater inhibition of amphetamine-induced hyperactivity. By comparison, administration of tacrine in combination with pimavanserin further resulted in inhibition for a particular dosage of tacrine. Thus, pimavanserin potentiates the ability of tacrine to suppress amphetamine-induced hyperactivity in mice. Figures 3 and 4 illustrate the total distance traveled as a function of xanomeline dosage and percent inhibition as a function of xanomeline dosage, respectively in mice treated with or without amphetamine. Similar results to tacrine as shown in Figures 1 and 2 were observed for xanomeline. These results demonstrated that pimavanserin enhanced the antipsychotic-like activity of cholinesterase inhibitors and muscarinic receptor agonists.

### Example 2 - Novel Object Recognition

Subjects were male, C57 BK/6 mice purchased from Harlan Laboratories, weighing 15-20g upon arrival. Animals were housed 8 per cage with food and water available *ad libitum.* Animals were housed on a 12 hr light cycle (lights on 6 am) for 4-7 days prior to behavioral testing.

Novel object recognition (NOR) was conducted in a novel environment consisting of a white plastic tub measuring 45.7 x 33.7 x 19 cm. Prior to each trial the bottom of the tub was covered with a piece of plastic lined bench top paper. There were two sets of identical objects chosen so that when given an opportunity to explore, mice would evenly divide exploration time between the objects. "A" objects were yellow, ceramic, 12-sided ramekins measuring 4 cm high x 7 cm diameter. "B" objects were 8 X 8 x 4 cm stainless steel, 4-sided ramekins.

At the beginning of each test day, animals were placed in groups of 6 into clean cages. Testing was conducted in three phases: acclimation, sample and test.

In the acclimation phase, each group of six mice was placed collectively into the NOR chamber and allowed to explore freely for 30 minutes. After acclimation animals were injected (dose and pretreatment time varied by test drug) and placed back into the cages to wait the pre-treatment interval.

During the sample phase, two identical objects ("A" or "B" objects described above) were placed into the NOR chamber. Objects were placed on diagonal corners of the long axis of the arena approximately 5 cm from the walls, while subjects were placed into one of the neutral corners (alternating across subjects). Each mouse was placed into the NOR chamber one at a time and was allowed to explore the chamber and the objects for 3 minutes. The time spent exploring at each position was recorded. Directly sniffing or touching the object was recorded as exploration. After 3 minutes, each mouse was removed from the arena and placed back into its cage.

The test phase was conducted one to two hours after the sample phase. During the test, one familiar object (seen during sample) and one novel object were placed into the chamber in the same positions used during the sample phase, and each mouse was allowed 3 minutes to explore. The test sessions were recorded on video and scored by an observer blind to each subject's treatment condition. Any time spent directly sniffing or touching an object was counted as exploration. The object serving as the novel object and the position where the novel object was placed were counterbalanced across subjects. Prior to each trial (acclimation, sample and test), all equipment was wiped with a Clorox wipe and bench paper (cut to fit) was placed in the bottom of the chamber.

In addition to time spent exploring each object (T_{N} = time spent exploring novel object, T_{F} = time spent exploring familiar object), two measures were determined for each subject: exploration ratio (% of time spent exploring at novel object) ER = T_{N}*100/(T_{N} + T_{F}) and discrimination index (preference for novel) DI = (T_{N}-T_{F})/(T_{N} + T_{F}).

During the sample phase, it was expected that there would be no preference for one position over another in any treatment condition. During the test phase, vehicle treated animals were expected to show a preference for the novel object after 1 hour (indicating they recognize the familiar object), but were expected to return to baseline exploration rates after 2 hours.

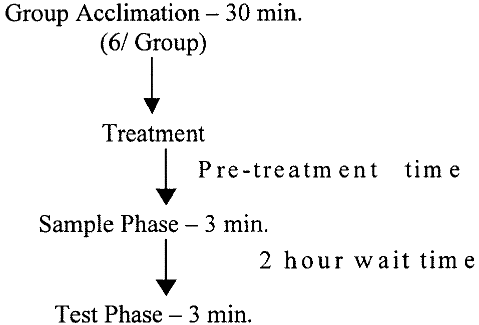

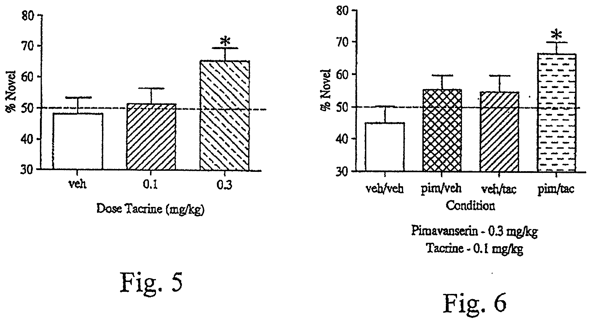

The results of these studies using tacrine are depicted in Figures 5 and 6. Figure 5 illustrates the percent of time each subject spent exploring a novel object as a function of the dosage of tacrine received. As the dosage of tacrine increased, the percent of time spent exploring a novel object also increased. Preference for exploring the novel object suggests that the subject recognizes and distinguishes that one object is novel, while the other object is familiar. Thus, Figure 5 demonstrates that tacrine improves the cognition of a subject.

Figure 6 compares novel object recognition data for four groups of subjects. Each group was treated with vehicle, pimavanserin, tacrine, or a combination of pimavanserin and tacrine. As shown in Figure 6, the percent of time spent exploring a novel object was highest for the group treated with a combination of pimavanserin and tacrine. Notably, this group performed better than the groups that had received either pimavanserin or tacrine individually. These findings demonstrate the ability of pimavanserin to augment the pro-cognitive effects of tacrine.

### Example 3 - Hyperactivity Study with an Amyloid Protein

This study was conducted in acrylic chambers (42 cm x 42 cm x 30 cm) equipped with 16 infrared photobeams along each horizontal axis (front-to-back and side-to-side, from Accuscan Instruments, Inc. (Columbus, OH)). Subjects were mice receiving ICV infusion of amyloid β protein fragment (25-35) or ICV infusion of saline (sham) for 7-10 days. This procedure has been shown to result in the formation of amyloid plaques and impaired cognition, thus simulating the effects of Alzheimer's disease.

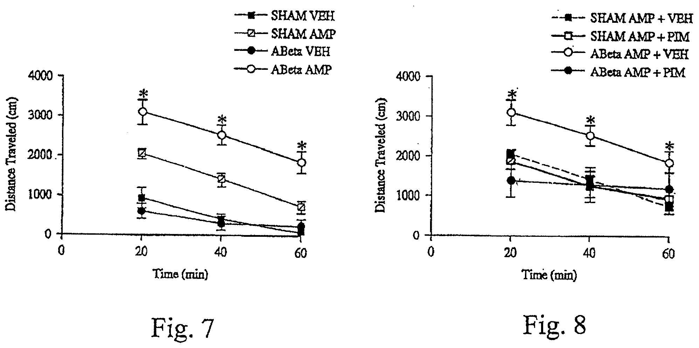

An amphetamine-induced hyperactivity study was conducted on the subjects. Subjects were injected with amphetamine (3 mg/kg) or vehicle 15 minutes prior to entering motor activity chambers. Activity was measured as a total distance traveled (DT) in centimeters (cm) and was determined in 20 minute increments across a 60 minute session. The results of this study are shown in Figure 7.

Figure 7 shows distance traveled as a function of time. Among the subjects injected with saline, amyloid β mice traveled the same distance over a given amount of time as the control animals; thus, these mice exhibited normal basal levels of locomotor activity. Among the subjects injected with amphetamine, amyloid β mice traveled farther on average than control mice. The findings demonstrate that amyloid β mice show an augmented response to amphetamine as compared to the control animals.

In a subsequent study, mice were pretreated with either pimavanserin (0.3 mg/kg) or vehicle 60 min prior to behavioral testing, and all subjects were subsequently injected with amphetamine (3 mg/kg) 15 minutes prior to entering motor activity chambers. Activity was measured as a total distance traveled (DT) in centimeters (cm) and was determined in 20 minute increments across a 60 minute session.

Figure 8 illustrates distance traveled as a function of time following amphetamine pretreatment. Among the subjects treated with vehicle, the amyloid β mice traveled farther than the control mice. In contrast, among the subjects treated with pimavanserin, subjects traveled similar distances regardless of whether they were in the amyloid β or control groups. Thus, in the control mice, pimavanserin alone did not reduce the hyperactivity induced by amphetamine. However, in a model of Alzheimer's disease model, pimavanserin reversed the augmented locomotor response to amphetamine. These results suggest that pimavanserin can be effective in treating, ameliorating, or preventing psychosis associated with Alzheimer's disease.

### Example 4 - Hyperactivity Study with M1 Knock-Out Mice

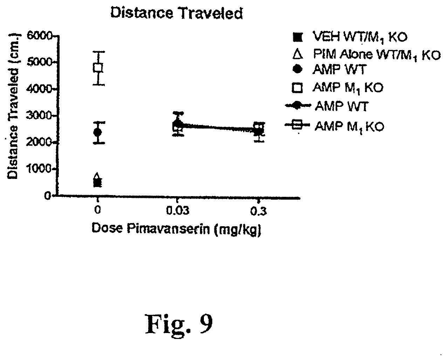

This study was conducted in acrylic chambers (42 cm x 42 cm x 30 cm) equipped with 16 infrared photobeams along each horizontal axis (front-to-back and side-to-side, from Accuscan Instruments, Inc. (Columbus, OH). Subjects were mice with a deletion of the M₁ muscarinic receptor (M₁KO) or wild-type (WT) controls. Vehicle (saline) or test compound (pimavanserin, 0.03 or 0.3 mg/kg) was injected 45 min prior to vehicle or amphetamine (3 mg/kg ip). Fifteen minutes later, mice were placed in the locomotor activity chambers and their activity was recorded. In order to generate dose-response curves, raw DT data were converted to %MPI: %MPI = ((DT drug - DT MK801 control)/(DT vehicle control - DT MK801control)) * 100. A linear regression analysis was then conducted to determine the theoretical dose that would produce a 50% reduction of activity (ED₅₀) and 95% confidence interval. Mice had no prior exposure to the chambers and each dose combination was tested in separate groups of mice. The results of these studies are presented in Figures 9-10.

Figure 9 illustrates the results of an amphetamine-induced hyperactivity study, showing distance traveled as a function of pimavanserin dosage. While not wanting to be bound by any particular theory, psychosis in humans can be associated with augmented dopamine in the striatum. Mice devoid of the muscarinic M1 receptors are hyperactive which can be correlated to augmented dopamine levels in the striatum. As shown in Figure 9, administration of pimavanserin alone had no significant effect on locomotor activity compared with vehicle controls. However, upon administration of amphetamine, M₁KO mice had an augmented locomotor response compared with WT controls. While pimavanserin did not alter amphetamine-induced activity in WT subjects, it reversed the augmented amphetamine response in M1KO subjects. Thus, after pimavanserin treatment, amphetamine produced a similar increase in activity in both WT and M1KO subjects.

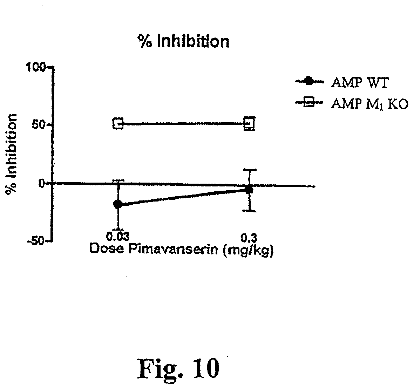

Figure 10 illustrates percent inhibition as a function of pimavanserin dosage generated from the distance traveled data shown in Figure 9. As demonstrated by Figure 10, pimavanserin increased the inhibition of amphetamine-induced hyperactivity. The results in Figures 9 and 10 indicate pimavanserin reduces augmented amphetamine-induced hyperactivity in mice devoid of muscarinic M1 receptors. Since reversal of amphetamine-induced hyperactivity is predictive of antipsychotic activity in humans, these data suggest that pimavanserin can be effective in treating or ameliorating psychosis associated with a deficiency in muscarinic receptor activity such as Alzheimer's disease-induced psychosis.

## Claims

1. A composition comprising:
a compound according to Formula (I) 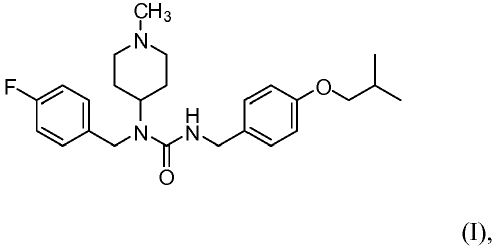 or a salt, or an isolated, substantially pure metabolite thereof; and
an agent that ameliorates one or more cholinergic abnormalities,
wherein the agent is a cholinesterase inhibitor selected from the group consisting of donepezil and tacrine.

2. The composition of Claim 1, wherein the cholinesterase inhibitor is tacrine.

3. The composition of Claim 1, wherein the cholinesterase inhibitor is donepezil.

4. The composition of any one of Claims 1 to 3, wherein the salt of a compound according to Formula (I) is a tartrate or hemi-tartrate salt.

5. The composition of any one of Claims 1 to 4 for use in ameliorating or treating a disease condition **characterized by** one or more cholinergic abnormalities, wherein the disease condition **characterized by** one or more cholinergic abnormalities is selected from the group consisting of a neuropsychiatric disorder, a neurodegenerative disorder, and an extrapyrimidal disorder.

6. The composition for use of Claim 5, wherein the neuropsychiatric disorder is selected from the group consisting of schizophrenia, a schizoaffective disorder, mania, depression, a cognitive disorder, aggressiveness, a panic attack, an obsessive compulsive disorder, borderline personality disorder, borderline disorder, multiplex developmental disorder (MDD), a behavioral disorder, psychosis, suicidal tendency, bipolar disorder, a sleep disorder, addiction, attention deficit hyperactivity disorder (ADHD), post traumatic stress disorder (PTSD), Tourette's syndrome, anxiety, autism, Down's syndrome, a learning disorder, a psychosomatic disorder, alcohol withdrawal, epilepsy, pain, a disorder associated with hypoglutamatergia, and serotonin syndrome.

7. The composition for use of Claim 5, wherein the neurodegenerative disorder is selected from the group consisting of Alzheimer's disease, Parkinson's disease, Huntington's chorea, sphinocerebellar atrophy, frontotemporal dementia, supranuclear palsy, or Lewy body dementia.

8. The composition for use of Claim 5, wherein the extrapyrimidal disorder is selected from the group consisting of dyskinesia, bradykinesia, rigidity, psychomotor slowing, tics, akathisia, Friedrich's ataxia, Machado-Joseph's disease, dystonia, tremor, restless legs syndrome, and myoclonus.

9. The composition for use of Claim 5, wherein said disease condition **characterized by** one or more cholinergic abnormalities is selected from the group consisting of cognitive impairment, forgetfulness, confusion, memory loss, an attention deficit disorder, depression, pain, psychosis, a hallucination, aggressiveness, and paranoia, wherein the psychosis is optionally selected from the group consisting of drug-induced psychosis, treatment-induced psychosis and psychosis associated with a disease.

10. The composition for use of Claim 5, wherein the disease is selected from the group consisting of dementia, post traumatic stress disorder, Alzheimer's disease, Parkinson's disease and schizophrenia.

## Patentansprüche

1. Zusammensetzung, umfassend:
eine Verbindung gemäß Formel (I) 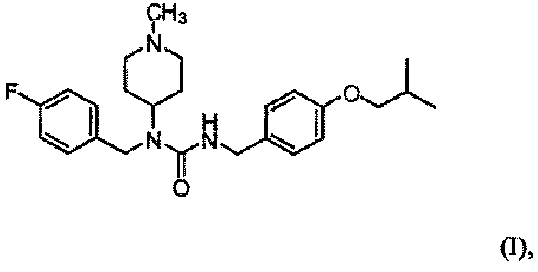 oder ein Salz oder einen isolierten, im Wesentlichen reinen Metaboliten davon und
ein Mittel, das eine oder mehrere cholinergene Anomalitäten lindert,
wobei es sich bei dem Mittel um einen aus der aus Donepezil und Tacrin bestehenden Gruppe ausgewählten Cholinesteraseinhibitor handelt.

2. Zusammensetzung nach Anspruch 1, wobei es sich bei dem Cholinesteraseinhibitor um Tacrin handelt.

3. Zusammensetzung nach Anspruch 1, wobei es sich bei dem Cholinesteraseinhibitor um Donepezil handelt.

4. Zusammensetzung nach einem der Ansprüche 1 bis 3, wobei es sich bei dem Salz einer Verbindung gemäß Formel (I) um ein Tartrat- oder Hemitartratsalz handelt.

5. Zusammensetzung nach einem der Ansprüche 1 bis 4 zur Verwendung bei der Linderung oder Behandlung eines durch eine oder mehrere cholinergene Anomalitäten gekennzeichneten Krankheitszustands, wobei der durch eine oder mehrere cholinergene Anomalitäten gekennzeichnete Krankheitszustand aus der aus einer neuropsychiatrischen Störung, einer neurodegenerativen Störung und einer extrapyrimidalen Störung bestehenden Gruppe ausgewählt ist.

6. Zusammensetzung zur Verwendung nach Anspruch 5, wobei die neuropsychiatrische Störung aus der aus Schizophrenie, einer schizoaffektiven Störung, Manie, Depression, einer kognitiven Störung, Aggressivität, einem Panikanfall, einer Zwangsneurose, einer Borderline-Persönlichkeitsstörung, einer Borderline-Störung, einer multiplexen Entwicklungsstörung (MDD), einer Verhaltensstörung, Psychose, Suizidneigung, bipolarer Störung, einer Schlafstörung, Sucht, Aufmerksamkeitsdefizit-/Hyperaktivitätsstörung (ADHD), posttraumatischer Belastungsstörung (PTSD), Tourette-Syndrom, Angst, Autismus, Down-Syndrom, einer Lernstörung, einer psychosomatischen Störung, Alkoholentzug, Epilepsie, Schmerzen, einer mit Hypoglutamatergie assoziierten Störung und einem Serotoninsyndrom bestehenden Gruppe ausgewählt ist.

7. Zusammensetzung zur Verwendung nach Anspruch 5, wobei die neurodegenerative Störung aus der aus Alzheimer-Krankheit, Parkinson-Krankheit, Chorea Huntington, spinozerebellärer Atrophie, Frontotemporaldemenz, supranukleärer Blickparese oder Lewy-Körperchen-Demenz bestehenden Gruppe ausgewählt ist.

8. Zusammensetzung zur Verwendung nach Anspruch 5, wobei die extrapyrimidale Störung aus der aus Dyskinesie, Bradykinesie, Spastizität, psychomotorischer Verlangsamung, Ticks, Akathisie, Friedreich-Ataxie, Machado-Joseph-Krankheit, Dystonie, Tremor, Restless-Legs-Syndrom und Myoklonie bestehenden Gruppe ausgewählt ist.

9. Zusammensetzung zur Verwendung nach Anspruch 5, wobei der durch eine oder mehrere cholinerge Anomalitäten gekennzeichnete Krankheitszustand aus der aus kognitiver Störung, Vergesslichkeit, Verwirrung, Gedächtnisverlust, einer Aufmerksamkeitsstörung, Depression, Schmerzen, Psychose, einer Halluzination, Aggressivität und Paranoia bestehenden Gruppe ausgewählt ist, wobei die Psychose gegebenenfalls aus der aus drogeninduzierter Psychose, behandlungsinduzierter Psychose und mit einer Krankheit assoziierten Psychose bestehenden Gruppe ausgewählt ist.

10. Zusammensetzung zur Verwendung nach Anspruch 5, wobei die Krankheit aus der aus Demenz, posttraumatischer Belastungsstörung, Alzheimer-Krankheit, Parkinson-Krankheit und Schizophrenie bestehenden Gruppe ausgewählt ist.

## Revendications

1. Composition comprenant :
un composé répondant à la Formule (I) 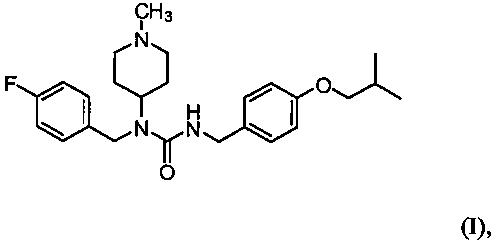
ou un sel, ou un métabolite isolé substantiellement pur de celui-ci ; et
un agent qui soulage une ou plusieurs anomalies cholinergiques ;
où l'agent est un inhibiteur de cholinestérase choisi dans le groupe constitue par le donépézil et la tacrine.

2. Composition selon la Revendication 1, où l'inhibiteur de cholinestérase est la tacrine.

3. Composition selon la Revendication 1, où l'inhibiteur de cholinestérase est le donépézil.

4. Composition selon l'une quelconque des Revendications 1 à 3, où le sel d'un composé selon la Formule (I) est un sel de tartrate ou d'hémi-tartrate.

5. Composition selon l'une quelconque des Revendications 1 à 4 pour utilisation dans le soulagement ou le traitement d'un état pathologique **caractérisé par** une ou plusieurs anomalies cholinergiques, où l'état pathologique **caractérisé par** une ou plusieurs anomalies cholinergiques est choisi dans le groupe constitué par un trouble neuropsychiatrique, un trouble neurodégénératif et un trouble extrapyramidal.

6. Composition pour utilisation selon la Revendication 5, où le trouble neuropsychiatrique est choisi dans le groupe constitué par les suivants : schizophrénie, trouble schizo-affectif, manie, dépression, trouble cognitif, agressivité, crise de panique, trouble obsessionnel compulsif, trouble de la personnalité limite, trouble limite, trouble atypique du développement, trouble du comportement, psychose, tendance suicidaire, trouble bipolaire, trouble du sommeil, addiction, trouble de déficit d'attention avec hyperactivité (TDAH), état de stress post-traumatique (ESPT), syndrome de Gilles de la Tourette, anxiété, autisme, syndrome de Down, troubles d'apprentissage, trouble psychosomatique, manque d'alcool, épilepsie, douleur, trouble associé à hypoglutamatergie, et syndrome sérotoninergique.

7. Composition pour utilisation selon la Revendication 5, où le trouble neurodégénératif est choisi dans le groupe constitué par les suivants : maladie d'Alzheimer, maladie de Parkinson, chorée de Huntington, atrophie spinocérébelleuse, démence fronto-temporale, paralysie supranucléaire ou démence à corps de Lewy.

8. Composition pour utilisation selon la Revendication 5, où le trouble extrapyramidal est choisi dans le groupe constitué par les suivants : dyskinésie, bradykinésie, rigidité, ralentissement psychomoteur, tics, akathisie, ataxie de Friedrich, maladie de Machado-Joseph, dystonie, tremblement, syndrome des jambes sans repos et myoclonie.

9. Composition pour utilisation selon la Revendication 5, où ledit état pathologique **caractérisé par** une ou plusieurs anomalies cholinergiques est choisi dans le groupe constitué par les suivants : déficit cognitif, amnésie, confusion, perte de mémoire, trouble de déficit d'attention, dépression, douleur, psychose, hallucination, agressivité et paranoïa, où la psychose est éventuellement choisie dans le groupe constitué par les suivantes : psychose induite par médicament, psychose induite par traitement et psychose associée à une maladie.

10. Composition pour utilisation selon la Revendication 5, où la maladie est choisie dans le groupe constitué par les suivantes : démence, état de stress post-traumatique, maladie d'Alzheimer, maladie de Parkinson et schizophrénie.
